(19) Europäisches Patentamt
European Patent Office
Office européen des brevets

(11) **EP 4 350 707 A1**

(12) **EUROPEAN PATENT APPLICATION**
published in accordance with Art. 153(4) EPC

(43) Date of publication:
**10.04.2024 Bulletin 2024/15**

(21) Application number: **22811703.2**

(22) Date of filing: **30.05.2022**

(51) International Patent Classification (IPC):
*G16H 50/20* (2018.01)    *G16B 30/10* (2019.01)
*G16B 40/20* (2019.01)    *G16H 50/50* (2018.01)
*G16H 30/40* (2018.01)    *G16H 30/20* (2018.01)
*G06N 3/08* (2006.01)    *G06N 3/04* (2006.01)

(52) Cooperative Patent Classification (CPC):
**G06N 3/04; G06N 3/08; G16B 30/10; G16B 40/20;
G16H 30/20; G16H 30/40; G16H 50/20;
G16H 50/50**

(86) International application number:
**PCT/KR2022/007648**

(87) International publication number:
**WO 2022/250512 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.05.2021 KR 20210068890**

(71) Applicant: **GC Genome Corporation
Yongin-si, Gyeonggi-do 16924 (KR)**

(72) Inventors:
• **CHOI, Jung Kyoon
Daejeon 34141 (KR)**
• **BAE, Min Gyun
Daejeon 34141 (KR)**
• **CHO, Eun Hae
Yongin-si Gyeonggi-do 16924 (KR)**
• **KI, Chang-Seok
Yongin-si Gyeonggi-do 16924 (KR)**

(74) Representative: **Mewburn Ellis LLP
Aurora Building
Counterslip
Bristol BS1 6BX (GB)**

(54) **ARTIFICIAL INTELLIGENCE-BASED METHOD FOR EARLY DIAGNOSIS OF CANCER, USING CELL-FREE DNA DISTRIBUTION IN TISSUE-SPECIFIC REGULATORY REGION**

(57)    The present invention relates to an artificial intelligence-based method for early diagnosis of cancer and, more specifically, to an artificial intelligence-based method for early diagnosis of cancer, using a method of inputting and analyzing information on cell-free DNA distribution in a tissue-specific regulatory region into an artificial intelligence model that has been trained to diagnose cancer early. The method for early diagnosis of cancer according to the present invention is high in commercial availability because it takes advantage of the information, obtained from the Next Generation Sequencing (NGS), on cell-free nucleic acid distribution in a tissue-specific regulatory region in early diagnosing cancer at high accuracy and sensitivity. Therefore, the method of the present invention is advantageous for early diagnosis of cancer.

EP 4 350 707 A1

FIG. 1

**Description**

[Technical Field]

The present invention relates to a cancer diagnosis method based on artificial intelligence and more specifically, to a cancer diagnosis method based on artificial intelligence including analyzing information of cell-free DNA distribution in a tissue-specific regulatory region input into an artificial intelligence model trained to perform early diagnosis of cancer.

[Background Art]

Research has been conducted on detection of chromosomal abnormalities by cell-free DNA (cfDNA) present in plasma by cell necrosis, apoptosis, and secretion using liquid biopsy. In particular, blood cell-free DNA derived from tumor cells includes tumor-specific chromosomal abnormalities and mutations that do not appear in normal cells, and has an advantage of showing the present state of tumors due to the half-life as short as 2 hours. In addition, cell-free DNA in blood is non-invasive and can be repeatedly collected and is in the spotlight as a tumor-specific biomarker in various cancer-related fields such as cancer diagnosis, monitoring, and prognosis.

Many researchers are making efforts to use liquid biopsy for early diagnosis using the advantage of the fact that cancer can be diagnosed only with a simple blood test. Since cancer is a disease caused by gradual accumulation of mutations in DNA, cancer-derived cfDNA is characterized by having mutations different from those of normal subjects and DNA containing mutations can be diagnosed as cancer using this characteristic. However, early cancer diagnosis using mutations has not yet exhibited excellent performance because there are very few mutations commonly found in cancer cells for different humans in the human genome, which consists of 3 billion copies, and there are many people who develop cancer even without those mutations.

Recently, a method including obtaining whole genome data of cfDNA, deriving a transcription start site profile based on read depth, and training the expression of each gene by SVM (Ulz, P., Thallinger, G., Auer, M. et al. Nat. Genet. Vol. 48, pp. 1273-1278, 2016), or a method for conducting early diagnosis of cancer or classifying cancer types by analyzing transcription factor binding patterns based on cfDNA fragmentation patterns (Ulz, P. et al., Nat. Commun. Vol. 10, 4666, 2019) has been developed, but this method has drawbacks of lower reliability or necessity of a large amount of data.

Under this technical background, as a result of diligent efforts to develop a method for early diagnosis of cancer based on artificial intelligence, the present inventors found that cancer can be diagnosed early with high sensitivity and accuracy by imaging the distribution of cell-free nucleic acids in tissue-specific regulatory regions and inputting the result to an artificial intelligence model trained to diagnose cancer early. Based thereon, the present invention was completed.

[Disclosure]

Therefore, it is one object of the present invention to provide a method for providing information for early diagnosis of cancer based on artificial intelligence.

It is another object of the present invention to provide a device for providing information for early diagnosis of cancer based on artificial intelligence.

It is another object of the present invention to provide a computer-readable storage medium including an instruction configured to be executed by a processor for providing information for early diagnosis of cancer.

It is another object of the present invention to provide a method for early diagnosis of cancer based on artificial intelligence.

It is another object of the present invention to provide a device for early diagnosis of cancer based on artificial intelligence.

It is another object of the present invention to provide a computer-readable storage medium including an instruction configured to be executed by a processor for conducting early diagnosis of cancer using the method.

In accordance with one aspect of the present invention, the above and other objects can be accomplished by the provision of a method for providing information for early diagnosis of cancer based on artificial intelligence, including: (a) obtaining a sequence information from extracted nucleic acids from a biological sample; (b) aligning the sequence information (reads) with a reference genome database; (c) selecting nucleic acid fragments of regulatory regions based on the aligned sequence reads; (d) producing image data from the selected nucleic acid fragments; and (e) inputting and analyzing the produced image data to an artificial intelligence model trained to distinguish a normal image from a cancer image, and then comparing an output value with a cut-off value to determine whether or not cancer develops.

In accordance with another aspect of the present invention, provided is a device for providing information for early diagnosis of cancer based on artificial intelligence, the device including: a decoder configured to extract nucleic acids from a biological sample and decode sequence information; an aligner configured to align the decoded sequence with a reference genome database; a nucleic acid fragment selector configured to select nucleic acid fragments of regulatory regions based on the aligned sequence reads; a data producer configured to produce the selected nucleic acid fragments as image data; and an information supply configured to input the produced image data to an artificial intelligence model trained to distinguish a normal image from a cancer image, to analyze the data, and to provide information for early diagnosis of cancer.

In accordance with another aspect of the present invention, provided is a computer-readable storage medium including an instruction configured to be executed by a processor for providing information for early diagnosis of cancer, through the following steps including: (a) obtaining a sequence information from extracted nucleic acids from a biological sample; (b) aligning the sequence information (reads) with a reference genome database; (c) selecting nucleic acid fragments of regulatory regions based on the aligned sequence reads; (d) producing image data from the selected nucleic acid fragments; and (e) inputting and analyzing the produced image data to an artificial intelligence model trained to distinguish a normal image from a cancer image, and then comparing an output value with a cut-off value to determine whether or not cancer develops.

In accordance with another aspect of the present invention, provided is a method for early diagnosis of cancer based on artificial intelligence, including: (a) obtaining a sequence information from extracted nucleic acids from a biological sample; (b) aligning the sequence information (reads) with a reference genome database; (c) selecting nucleic acid fragments of regulatory regions based on the aligned sequence reads; (d) producing image data from the selected nucleic acid fragments; and (e) inputting and analyzing the produced image data to an artificial intelligence model trained to distinguish a normal image from a cancer image, and then comparing an output value with a cut-off value, and determining that cancer develops when the output value is higher than the cut-off value.

In accordance with another aspect of the present invention, provided is a device for early diagnosis of cancer based on artificial intelligence, the device including: a decoder configured to extract nucleic acids from a biological sample and decode sequence information; an aligner configured to align the decoded sequence with a reference genome database; a nucleic acid fragment selector configured to select nucleic acid fragments of regulatory regions based on the aligned sequence reads; a data producer configured to produce the selected nucleic acid fragments as image data; and a cancer diagnostic unit configured to input the produced image data to an artificial intelligence model trained to distinguish a normal image from a cancer image, to compare an output value with a cut-off value and to determine that cancer develops when the output value is higher than the cut-off value.

In accordance with another aspect of the present invention, provided is a computer-readable storage medium including an instruction configured to be executed by a processor for conducting early diagnosis of cancer, through the following steps including: (a) obtaining a sequence information from extracted nucleic acids from a biological sample; (b) aligning the sequence information (reads) with a reference genome database; (c) selecting nucleic acid fragments of regulatory regions based on the aligned sequence reads; (d) producing image data from the selected nucleic acid fragments; and (e) inputting and analyzing the produced image data to an artificial intelligence model trained to distinguish a normal image from a cancer image, and then comparing an output value with a cut-off value, and determining that cancer develops when the output value is higher than the cut-off value.

[Description of Drawings]

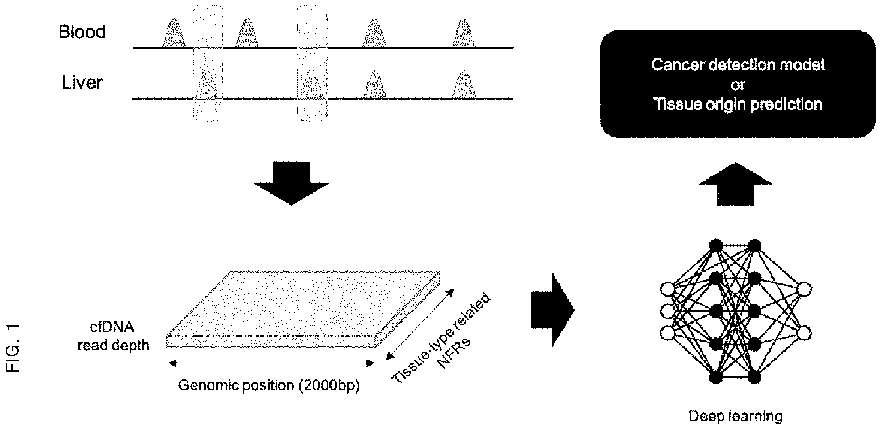

FIG. 1 is an overall flowchart for implementing the method of the present invention.

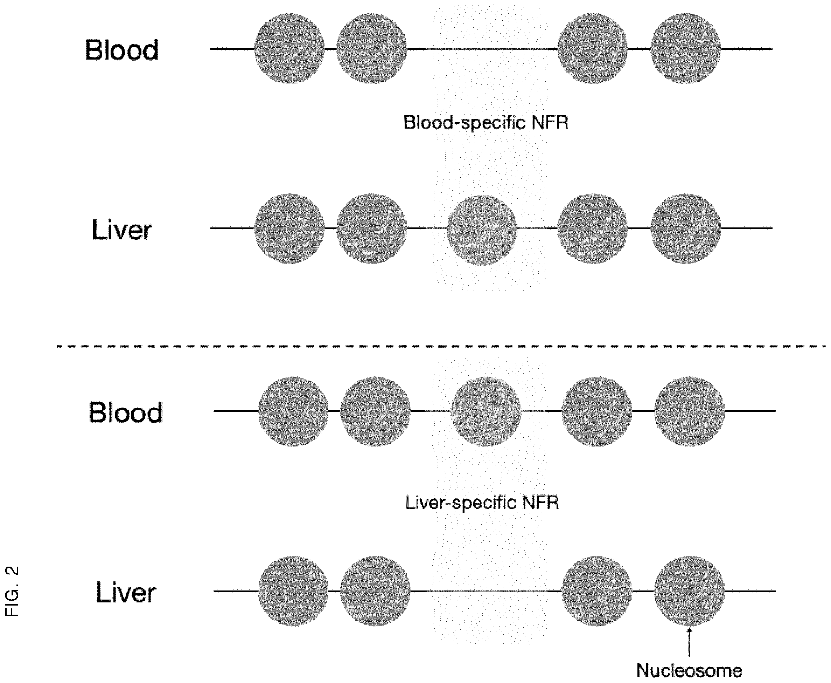

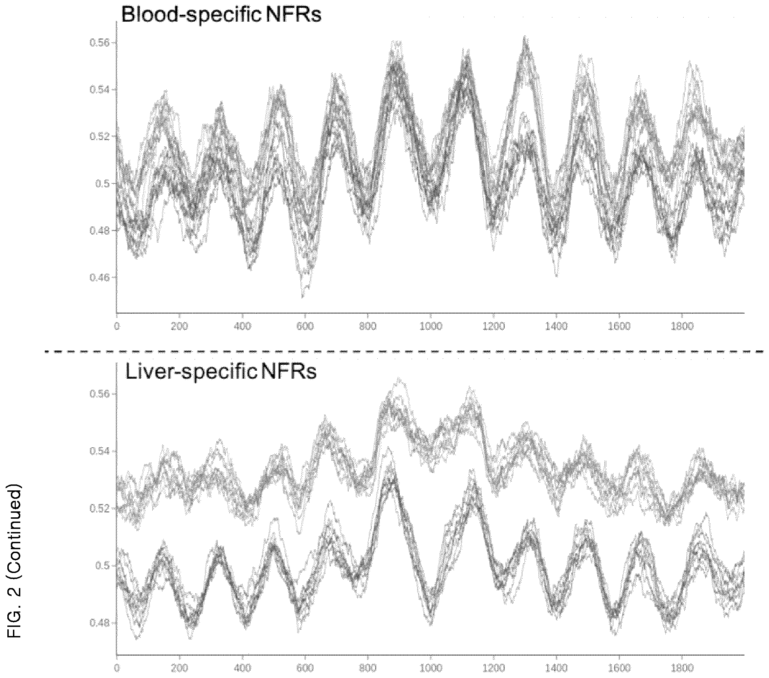

FIG. 2 is a schematic diagram and an actual example illustrating the difference in nucleosome position in the regulatory region by tissue.

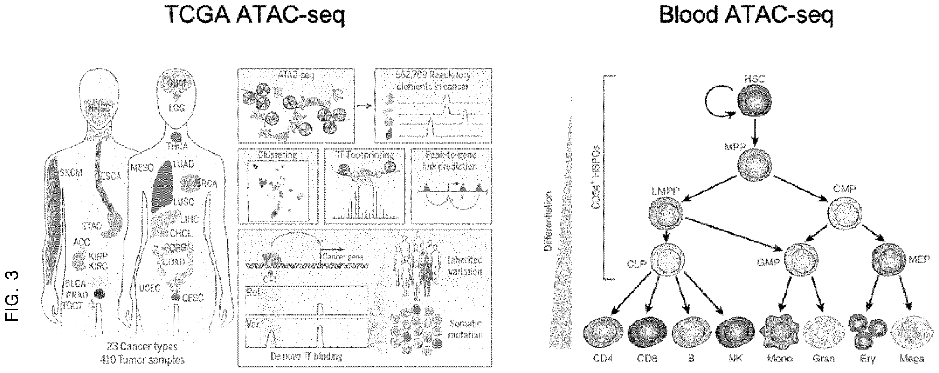

FIG. 3 is a schematic diagram illustrating modulator data for various tissues.

FIG. 4 is a schematic diagram illustrating a method for discovering tissue-specific modulators.

FIG. 5 shows the principle of producing image data from the cfDNA distribution of the regulatory region obtained according to an embodiment of the present invention to input the cfDNA distribution to an artificial intelligence model.

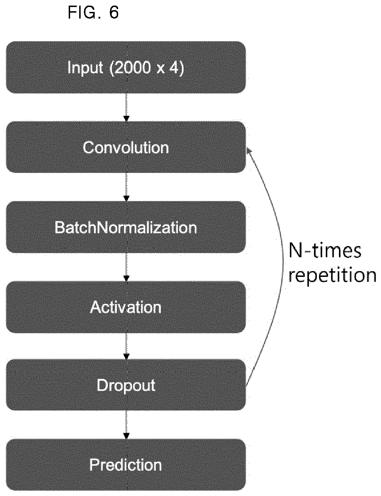

FIG. 6 illustrates an algorithm of an artificial intelligence model constructed according to an embodiment of the present invention.

FIG. 7 shows the result of the performance of a liver cancer prediction model constructed according to an embodiment of the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

Terms such as first, second, A, B, and the like may be used to describe various elements, but these elements are not limited by these terms and are merely used to distinguish one element from another. For example, without departing from the scope of the technology described below, a first element may be referred to as a second element and in a similar way, the second element may be referred to as a first element. "And/or" includes any combination of a plurality of related recited items or any one of a plurality of related recited items.

Singular forms are intended to include plural forms as well, unless the context clearly indicates otherwise. It will be further understood that the terms "comprises" and/or "comprising", when used in this specification, specify the presence of features, numbers, steps, actions, components, parts, or combinations thereof, but do not preclude the presence or addition of one or more other features, numbers, steps, actions, components, parts, or combinations thereof.

Prior to the detailed description of the drawings, it is clear that the classification of components in the present specification is merely made depending on the main function of each component. That is, two or more components described below may be combined into one component or one component may be divided into two or more depending on each more detailed function. In addition, each component to be described below may further perform some or all of the functions of other components in addition to its main function, and some of the main functions of each component may be performed exclusively by other components.

In addition, in implementing a method or operation method, respective steps constituting the method may occur in a different order from a specific order unless the specific order is clearly described in context. That is, the steps may be performed in the specific order, substantially simultaneously, or in reverse order to that specified.

The present invention is intended to diagnose cancer early with high sensitivity and accuracy by aligning sequencing data obtained from a sample with a reference genome database, selecting nucleic acid fragments of regulatory regions based on the aligned sequence reads, producing image data from the selected nucleic acid fragments, and inputting the produced image data to an artificial intelligence model trained to distinguish a normal image from a cancer image.

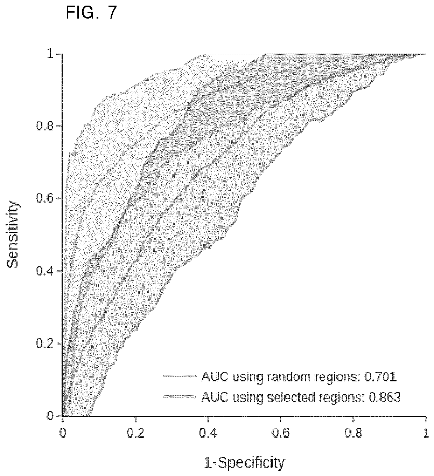

That is, in one embodiment of the present invention, nucleic acids were extracted from liquid biopsies obtained from 187 normal subjects, 12 early liver cancer patients, and 150 late liver cancer patients, cfDNA sequencing was performed to select nucleic acid fragments corresponding to liver-specific regulatory regions, image data was produced from the nucleic acid fragments, an artificial intelligence training model for early diagnosis of liver cancer was constructed using image data of 187 normal subjects and 150 patients with early liver cancer, and the performance of the training model was evaluated using the data of 12 patients with early liver cancer. The results showed that the training model constructed with high accuracy could discriminate normal subject images from liver cancer patient and early liver cancer patient images (FIGS. 7 and 8).

As used herein, the term "read" refers to a single nucleic acid fragment, sequence information of which is analyzed using various methods known in the art. Therefore, the terms "sequence information" and "read" have the same meaning in that both are sequence information obtained through a sequencing process.

As used herein, the term "regulatory region" refers to any position of the chromosome where gene expression can be regulated, and refers to a region where an RNA synthetase and a transcriptional regulation protein bind for RNA synthesis. Preferably, the regulatory region may include a promoter, enhancer, silencer, and insulator, but is not limited thereto.

As used herein, the term "NFR (nucleosome free region)" refers to the same region as the regulatory region, but specifically refers to an area of the regulatory region where a nucleosome does not exist. For example, in an enhancer region including a first nucleosome of 1 to 147 bp, a nucleic acids between nucleosomes of 148 to 364 bp, a second nucleosome of 347 to 493 bp, a nucleic acids between nucleosomes of 494 to 692 bp, a third nucleosome of 693 to 839 bp, and a nucleic acids between nucleosomes of 840 to 1,039 bp, when transcription is initiated, the second nucleosome is released, and the transcription regulatory protein is bound, the NFR corresponds to a 148 to 692 bp region.

In addition, although transcription proceeds in normal samples in the same manner as above, NFRs may not be present in cancer samples, nucleosomes of other regions are released and thus other NFRs may be formed, or NFRs that do not exist in normal samples may be newly generated in cancer samples.

In addition, although transcription proceeds in the same manner as above in blood cells, but NFR may not be present in other tissues (e.g., liver), nucleosomes of other regions may be released and thus other NFRs may be produced, or NFRs that do not exist in blood samples may be newly generated in cancer samples.

In another aspect, the present invention is directed to a method for providing information for early diagnosis of cancer based on artificial intelligence, including:

(a) obtaining a sequence information from extracted nucleic acids from a biological sample;

(b) aligning the sequence information (reads) with a reference genome database;

(c) selecting nucleic acid fragments of regulatory regions based on the aligned sequence reads;

(d) producing image data from the selected nucleic acid fragments; and

(e) inputting and analyzing the produced image data to an artificial intelligence model trained to distinguish a normal image from a cancer image, and then comparing an output value with a cut-off value to determine whether or not cancer develops.

In the present invention, the cancer may be a solid cancer or a blood cancer, is preferably selected from the group consisting of non-Hodgkin lymphoma, Hodgkin lymphoma, acute-myeloid leukemia, acute-lymphoid leukemia, multiple myeloma, head and neck cancer, lung cancer, glioblastoma, colorectal/rectal cancer, pancreatic cancer, breast cancer, ovarian cancer, melanoma, prostate cancer, thyroid cancer, liver cancer, stomach cancer, gallbladder cancer, biliary tract cancer, bladder cancer, small intestine cancer, cervical cancer, cancer of unknown primary, kidney cancer, and mesothelioma, and is most preferably liver cancer, but the cancer is not limited thereto.

In the present invention, step (a) to obtain sequence information includes:

(a-i) obtaining nucleic acids from a biological sample;
(a-ii) removing proteins, fats, and other residues from the obtained nucleic acids using a salting-out method, a column chromatography method, or a bead method to obtain purified nucleic acids;
(a-iii) producing a single-end sequencing or paired-end sequencing library for the purified nucleic acids or nucleic acids randomly fragmented by enzymatic digestion, pulverization, or hydroshearing;
(a-iv) reacting the produced library with a next-generation sequencer; and
(a-v) obtaining sequence information (reads) of the nucleic acids in the next-generation sequencer.

In the present invention, the step (a) to obtain sequence information may include obtaining the isolated cell-free DNA through whole genome sequencing at a depth of 1 million to 100 million reads.

In the present invention, the biological sample refers to any substance, biological fluid, tissue or cell obtained from or derived from a subject, and examples thereof include, but are not limited to, whole blood, leukocytes, peripheral blood mononuclear cells, leukocyte buffy coat, blood including plasma and serum, sputum, tears, mucus, nasal washes, nasal aspirates, breath, urine, semen, saliva, peritoneal washings, pelvic fluids, cystic fluids, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, semen, hair, saliva, urine, oral cells, placental cells, cerebrospinal fluid, and mixtures thereof.

As used herein, the term "reference population" refers to a reference group that is used for comparison like a reference genome database and refers to a population of subjects who do not currently have a specific disease or condition. In the present invention, the reference nucleotide sequence in the reference genome database of the reference population may be a reference chromosome registered with public health institutions such as the NCBI.

In the present invention, the nucleic acid in step (a) may be cell-free DNA, more preferably circulating tumor DNA, but is not limited thereto.

In the present invention, the next-generation sequencer may be used for any sequencing method known in the art. Sequencing of nucleic acids isolated using the selection method is typically performed using next-generation sequencing (NGS). Next-generation sequencing includes any sequencing method that determines the nucleotide sequence either of each nucleic acid molecule or a proxy cloned from each nucleic acid molecule so as to be highly similar thereto (e.g., 105 or more molecules are sequenced simultaneously). In one embodiment, the relative abundance of nucleic acid species in the library can be estimated by counting the relative number of occurrences of the sequence homologous thereto in data produced by sequencing experimentation. Next-generation sequencing is known in the art, and is described, for example, in Metzker, M. (2010), Nature Biotechnology Reviews 11:31-46, which is incorporated herein by reference.

In one embodiment, next-generation sequencing is performed to determine the nucleotide sequence of each nucleic acid molecule (using, for example, a HelioScope Gene-Sequencing system from Helicos Biosciences or a PacBio RS system from Pacific Biosciences). In other embodiments, massive parallel short-read sequencing, which produces more bases of the sequence per sequencing unit than other sequencing methods, for example, other sequencing methods that produce fewer but longer reads, determines the nucleotide sequence of a proxy cloned from each nucleic acid molecule (using, for example, a Solexa sequencer from Illumina Inc., located in San Diego, CA; 454 Life Sciences (Branford, Connecticut) and Ion Torrent). Other methods or devices for next-generation sequencing may be provided by 454 Life Sciences (Branford, Connecticut), Applied Biosystems (Foster City, CA; SOLiD Sequencer), Helicos Biosciences Corporation (Cambridge, MA) and emulsion and microfluidic sequencing nanodrops (e.g., GnuBIO Drops), but are not limited thereto.

Platforms for next-generation sequencing include, but are not limited to, the FLX System genome sequencer (GS) from Roche/454, the Illumina/Solexa genome analyzer (GA), the Support Oligonucleotide Ligation Detection (SOLiD) system from Life/APG, the G.007 system from Polonator, the HelioScope gene-sequencing system from Helicos Biosciences, and the PacBio RS system from Pacific Biosciences.

In the present invention, the alignment of step (b) may be performed using the BWA algorithm and the Hg19 sequence, but is not limited thereto.

In the present invention, the BWA algorithm may include BWA-ALN, BWA-SW or Bowtie2, but is not limited thereto.

In the present invention, the method may further include selecting reads having a mapping quality score of the aligned nucleic acid fragments equal to or greater than a cut-off value prior to step (c), wherein any value capable of confirming the quality of the aligned nucleic acid fragments may be used as the cut-off value without limitation and the cut-off value is preferably 50 to 70, more preferably 60, but is not limited thereto.

In the present invention, the regulatory region of step (c) may be a tissue-specific regulatory region.

In the present invention, the tissue-specific regulatory region may be characterized in that the length and/or amount of cell-free DNA detected for respective tissues is different.

In the present invention, in the tissue-specific regulatory region, the lengths and/or amounts of cell-free DNA detected only in specific tissues, for example, in the liver, are different from the lengths and/or amounts of cell-free DNA detected in other tissues, for example, blood, brain, stomach and heart, or the lengths and/or amounts of cell-free DNA detected in solid tissues (brain, liver, stomach, lungs, heart and the like) are different from the lengths and/or amounts of cell-free DNA detected in blood tissues (blood cells, bone marrow and the like).

In the present invention, the tissue-specific regulatory region may more specifically mean a region of the regulatory region where a nucleosome does not exist, that is, a nucleosome free Region (NFR), but is not limited thereto.

In the present invention, the number of tissue-specific regulatory region is not limited as long as image data input to the artificial intelligence model can be produced, and is preferably 10, 100, 1,000, 10,000, 20,000, or 50,000, but is not limited thereto.

In the present invention, the image in step (d) may be used without limitation as long as it can be used to train the artificial intelligence model, and is preferably a one-dimensional image wherein the x-axis is composed of the number of reads for each alignment position of the selected nucleic acid fragment, but is not limited thereto.

In the present invention, the image in step (d) is created from a list of values of cfDNA reads accumulated for each base pair and may have a structure in the form of, for example, [0.91, 0.93, ~~ , 0.73, 0.86], when ±1000 bp, namely, a total of 2,000 bp, is based on the position selected as the tissue-specific regulatory region, the number in [ ] becomes 2,000.

In the present invention, any artificial intelligence model in step (e) may be used without limitation, as long as it is an intelligence model trained to distinguish a normal image from a cancer image, is preferably an artificial neural network and is more preferably selected from the group consisting of a convolutional neural network (CNN), or a recurrent neural network (RNN), but is not limited thereto.

In the present invention, the reference value in step (e) can be used without limitation as long as it is used for early diagnosis of cancer and is preferably 0.5, but is not limited thereto, and when the reference value is 0.5, it is determined that cancer develops when the output value is 0.5 or more.

In the present invention, the artificial intelligence model is trained to adjust an output value to about 1 if there is cancer and to adjust an output value to about 0 if there is no cancer. Therefore, performance (training, validation, test accuracy) is measured based on a cut-off value of 0.5. In other words, if the output value is 0.5 or more, it is determined that there is cancer, and if it is less than 0.5, it is determined that there is no cancer.

Here, it will be apparent to those skilled in the art that the cut-off value of 0.5 may be arbitrarily changed. For example, in an attempt to reduce false positives, the cut-off value may be set to be higher than 0.5 as a stricter criterion for determining whether or not there is cancer, and in an attempt to reduce false negatives, the cut-off value may be set to be lower than 0.5 as a weaker criterion for determining that there is cancer.

In the present invention, when the artificial intelligence model is a CNN, a loss function is represented by Equation 1 below:

Equation 1:

$$\text{loss}(\text{model}(x), y) = -\frac{1}{n}\left[\sum_{i=1}^{n}\left(y_i \log\left(model(x_i)\right) + (1 - y_i)\log\left(1 - model(x_i)\right)\right)\right]$$

wherein N represents the number of training data, y represents an actual label value, and p(y) represents the probability value predicted through the model.

In the present invention, when the artificial intelligence model is a DNN, the training includes the following steps:

i) classifying the detected mutation data into training, validation, and test data,
wherein the training data is used to train the artificial intelligence model, the validation data is used to validate hyper-parameter tuning, and the test data is used for the test after optimal model production; and
ii) constructing an optimal artificial intelligence model through hyper-parameter tuning and training; and
iii) comparing the performance of multiple models obtained through hyper-parameter tuning using the validation data and determining the model having the best validation data as the optimal model.

In the present invention, hyper-parameter tuning is a process of optimizing the values of various parameters (the number of convolution layers, the number of dense layers, the number of convolution filters, etc.) constituting the artificial intelligence model. Hyper-parameter tuning is performed using Bayesian optimization and grid search methods.

In the present invention, the internal parameters (weights) of the artificial intelligence model are optimized using predetermined hyper-parameters, and it is determined that the model is over-fit when validation loss starts to increase compared to training loss and then training is stopped.

In the present invention, any value resulting from analysis of the image data input to the artificial intelligence model in step (e) may be used without limitation, as long as it is a specific score or real number, and the value is preferably a real number, but is not limited thereto.

In the present invention, the real number means a value expressed as a probability value by adjusting the output of the artificial intelligence model to a scale of 0 to 1 using the sigmoid function or SoftMax function for the last layer.

In another aspect, the present invention is directed to a device for providing information for early diagnosis of cancer based on artificial intelligence, the device including:

a decoder configured to extract nucleic acids from a biological sample and decode sequence information;

an aligner configured to align the decoded sequence with a reference genome database;

a nucleic acid fragment selector configured to select nucleic acid fragments of regulatory regions based on the aligned sequence reads;

a data producer configured to produce the selected nucleic acid fragments as image data; and

an information supply configured to input the produced image data to an artificial intelligence model trained to distinguish a normal image from a cancer image, and to provide information for early diagnosis of cancer.

In the present invention, the decoder may include a nucleic acid injector configured to inject the nucleic acid extracted from an independent device, and a sequence information analyzer configured to analyze the sequence information of the injected nucleic acid, preferably an NGS analyzer, but is not limited thereto.

In the present invention, the decoder may receive and decode sequence information data generated in the independent device.

In another aspect, the present invention is directed to a computer-readable storage medium including an instruction configured to be executed by a processor for providing information for early diagnosis of cancer, through the following steps including:

(a) obtaining a sequence information from extracted nucleic acids from a biological sample;

(b) aligning the sequence information (reads) with a reference genome database;

(c) selecting nucleic acid fragments of regulatory regions based on the aligned sequence reads;

(d) producing image data from the selected nucleic acid fragments; and

(e) inputting and analyzing the produced image data to an artificial intelligence model trained to distinguish a normal image from a cancer image, and then comparing an output value with a cut-off value to determine whether or not cancer develops.

In another aspect, the method according to the present disclosure may be implemented using a computer. In one embodiment, the computer includes one or more processors coupled to a chipset. In addition, a memory, a storage device, a keyboard, a graphics adapter, a pointing device, a network adapter and the like are connected to the chipset. In one embodiment, the performance of the chipset is acquired by a memory controller hub and an I/O controller hub. In another embodiment, the memory may be directly coupled to a processor instead of the chipset. The storage device is any device capable of maintaining data, including a hard drive, compact disc read-only memory (CD-ROM), DVD, or other memory devices. The memory relates to data and instructions used by the processor. The pointing device may be a mouse, track ball or other type of pointing device, and is used in combination with a keyboard to transmit input data to a computer system. The graphics adapter presents images and other information on a display. The network adapter is connected to the computer system through a local area network or a long distance communication network. However, the computer used herein is not limited to the above configuration, may not have some configurations, may further include additional configurations, and may also be part of a storage area network (SAN), and the computer of the present invention may be configured to be suitable for the execution of modules in the program for the implementation of the method according to the present invention.

The module used herein may mean a functional and structural combination of hardware to implement the technical idea according to the present invention and software to drive the hardware. For example, it will be apparent to those skilled in the art that the module may mean a logical unit of predetermined code and a hardware resource to execute the predetermined code, and does not necessarily mean physically connected code or one type of hardware.

In another aspect, the present invention is directed to a method for early diagnosis of cancer based on artificial intelligence, including:

(a) obtaining a sequence information from extracted nucleic acids from a biological sample;

(b) aligning the sequence information (reads) with a reference genome database;

(c) selecting nucleic acid fragments of regulatory regions based on the aligned sequence reads;

(d) producing image data from the selected nucleic acid fragments; and

(e) inputting and analyzing the produced image data to an artificial intelligence model trained to distinguish a normal image from a cancer image, and then comparing an output value with a cut-off value, and determining that cancer develops when the output value is higher than the cut-off value.

In another aspect, the present invention is directed to a method of treating a cancer patient including: (a) inputting the nucleic acid fragment image data of the regulatory region into an artificial intelligence model using the method and analyzing the data; (b) determining that cancer is present when a value output from the artificial intelligence model is higher than the cut-off value; and (c) treating a patient determined to have cancer.

In the present invention, the cancer therapy may be used without limitation as long as it can treat cancer or microscopic residual cancer and is preferably performed with one or more selected from the group consisting of surgery, adjuvant chemotherapy, neoadjuvant chemotherapy, radiation therapy, hormone therapy, cytotoxic therapy, immunotherapy, adaptive T cell therapy, targeted therapy, and combinations thereof, is more preferably performed by administering a cancer therapeutic agent, and is most preferably performed by administering one or more anticancer-agents selected from the group consisting of chemotherapy agents, targeted anticancer agents, and immunotherapeutic agents, but is not limited thereto.

In another aspect, the present invention is directed to a device for providing information for early diagnosis of cancer based on artificial intelligence, the device including: a decoder configured to extract nucleic acids from a biological sample and decode sequence information; an aligner configured to align the decoded sequence with a reference genome database; a nucleic acid fragment selector configured to select nucleic acid fragments of regulatory regions based on the aligned sequence reads; a data producer configured to produce the selected nucleic acid fragments as image data; and a cancer diagnostic unit configured to input the produced image data to an artificial intelligence model trained to distinguish a normal image from a cancer image, to compare an output value with a cut-off value, and to determine that cancer develops when the output value is higher than the cut-off value.

In another aspect, the present invention is directed to a computer-readable storage medium including an instruction configured to be executed by a processor for conducting early diagnosis of cancer, through the following steps including: (a) obtaining a sequence information from extracted nucleic acids from a biological sample; (b) aligning the sequence information (reads) with a reference genome database; (c) selecting nucleic acid fragments of regulatory regions based on the aligned sequence reads; (d) producing image data from the selected nucleic acid fragments; and (e) inputting and analyzing the produced image data to an artificial intelligence model trained to distinguish a normal image from a cancer image, and then comparing an output value with a cut-off value, and determining that cancer develops when the output value is higher than the cut-off value.

Example

Hereinafter, the present invention will be described in more detail with reference to examples. However, it will be obvious to those skilled in the art that these examples are provided only for illustration of the present invention, and should not be construed as limiting the scope of the present invention.

**Example 1. Identification of regulatory regions**

Regulatory regions may be identified by next generation sequencing (NGS) such as ATAC-seq, DNase-seq, and FAIRE-seq. The present inventors used TCGA data, which produced regulatory region data for over 400 patients for 23 cancer types, and data that profiled regulatory regions for 16 blood cells (DOI: 10.1126/science.aav1898, DOI:https://doi.org/10.1038/ng.3646).

A tool called "HMMRATAC" was used to find the nucleosome free regions (NFRs) using the corresponding regulator data and the MACS2 tool was used to find the regulatory regions. HMMRATAC found NFRs on the genome using the default option and MACS2 tool used the "--shift -75-extsize 150 --nomodel --nolambda --call-summits -q 0.05 -B - SPMR" option to find regulatory regions.

First, 39,604 NFRs for B cells, 40,795 NFRs for CD4 T cells, 44,687 NFRs for CD8 T cells, 36,342 NFRs for monocytes, and 42,458 NFRs for NK cells were found using HMMRATAC. Thereamong, CD8 T cells, which had the highest number of NFRs, was used as a representative blood cell type, and whether or not the NFR regions found from CD8 T cells corresponded to the regulatory regions of 17 liver cancer patients was determined using intersectBed of Bedtools. At this time, since the default option of intersectBed was used, if more than half of the two regions overlapped, it was determined that they overlapped with each other.

Similarly, using the ATAC-seq data of 17 liver cancer patients, NFR regions were found with the HMMRATAC program, and at least 13,712 to 62,344 NFR regions were obtained for each sample. Thereamong, the sample having the largest number of 62344 NFRs was used as representative liver cancer, and overlapped with a total of 5 blood cell types and calculation was performed in the same manner as above. When NFRs of CD8 T cells, a representative blood cell type, overlapped with liver cancer regulatory regions, portions of NFRs of CD8 T cells that did not overlap with the liver cancer regulatory regions were defined as "blood-specific NFRs", and portions of NFRs that entirely overlapped with liver cancer regulatory regions were defined "blood common NFRs". On the other hand, when NFRs of representative liver cancer samples overlapped with blood cell regulatory regions, portions of NFRs of representative liver cancer sample that did not overlap with the blood cell regulatory regions were defined as "liver cancer-specific NFRs", and portions of NFRs that entirely overlapped with blood-specific NFRs is defined "liver cancer common NFRs".

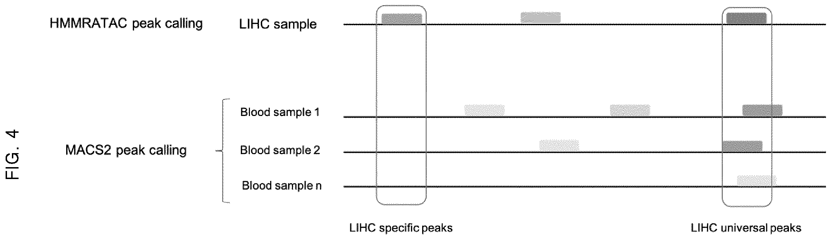

8,806 blood cell-specific NFRs, 17,508 blood common NFRs, 24,642 liver cancer-specific NFRs, and 19,134 liver cancer common NFRs were selected using this method and a deep learning image was constructed from the distribution of cfDNA reads accumulated in these regions (FIG. 4).

**Example 2. Construction of artificial intelligence model**

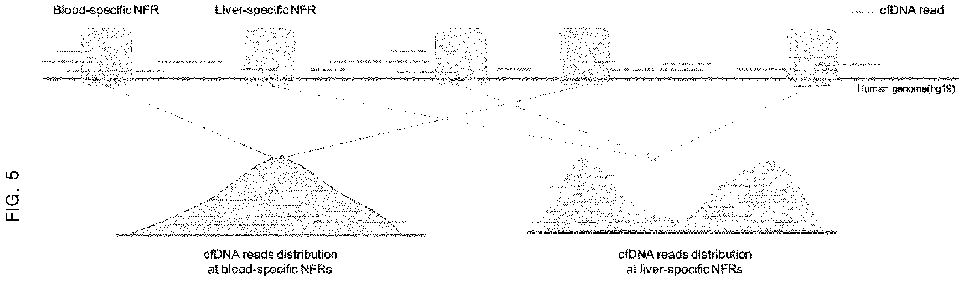

The distribution of cfDNA used as an input for a deep learning model at the location of the regulatory region was produced as shown in FIG. 5.

In other words, information on millions of cfDNA fragments floating around in the blood can be obtained through NGS, and information on the location of cfDNA fragments in the genome is accumulated on the x-axis to form a 1D image using each cfDNA fragment located in the regulatory region (FIG. 5).

Deep learning input images of tissue-specific regulatory regions were created. At this time, two input images of blood cell-specific regulatory regions and liver cancer-specific regulatory regions were created and then combined to form the final image, since the model is a model that distinguishes between normal subjects and liver cancer patients.

As the position of cfDNA corresponding to the x-axis, the area corresponding to $\pm$ 1,000 bp from the center of the NFR called "HMMRATAC", that is, a total of 2,000 bp was used. That is, a 1D image was constructed from the values of the accumulated cfDNA reads for each bp.

Therefore, the final input image consists of 2,000 (x axis, the position of cfDNA) × 4 (blood cell-specific, common regulatory region, liver cancer-specific, common regulatory region).

The convolutional neural network (CNN) model exhibits excellent performance in image classification because it exhibits local features well through the kernel. The cfDNA distribution was generated as image data, a pattern was trained with a CNN model, and a model for determining whether cancer develops or a normal state is maintained was created using the trained pattern.

**Experimental Example 1. Construction of liver cancer early diagnosis model**

To determine whether or not this model can be used for liver cancer diagnosis, blood was collected from 187 healthy subjects and 64 liver cancer patients and stored in Streck tubes. After centrifugation, the plasma on top of the blood was separated and cfDNA was extracted using the Tiangen kit and then sequenced using MGI DNB-seq.

A total of 251 people with advanced liver cancer patients and healthy subjects were used for model training, 150 subjects were used for training, 49 subjects were trained for validation, and performance of 52 subjects was evaluated with 52 people as a test.

In deep learning, as the amount of training data increases, the performance of the training is improved. In order to increase the number of samples for training, down-sampling was performed on each sample and $1.7 \times 10^7$ reads were randomly selected 10 times to increase the number of samples.

[Table 1]

|  | Model training | | | total sample |
|---|---|---|---|---|
|  | train | validation | test |  |
| hcc | 38 | 12 | 14 | 64 |
| healthy | 112 | 37 | 38 | 187 |

**Experimental Example 2. Evaluation of liver cancer early diagnosis model performance**

Various hyperparameters were tuned using Hyperband using 2,020 training sets, 670 validation sets, and 680 test sets, and finally, high performance was obtained as AUC of 0.98 in training, AUC of 0.94 in validation, and AUC of 0.86 in test (FIG. 7).

In addition, when a randomly selected region rather than the tissue-specific NFR selected above was used, it was found that AUC was 0.83 in training, 0.79 in validation, and 0.70 in test, which indicates that the selected tissue-specific NFR is important in distinguishing between normal subjects and liver cancer patients, and liver cancer patients are accurately selected through the selected regions.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

[Industrial applicability]

The early cancer diagnosis method according to the present invention is highly industrially applicable and is thus useful for early cancer diagnosis because it provides early diagnosis for cancer with high accuracy and sensitivity based on artificial intelligence using distribution of cell-free nucleic acids in tissue-specific regulatory regions through next generation sequencing (NGS).

**Claims**

1. A method for providing information for early diagnosis of cancer based on artificial intelligence, comprising:

   (a) obtaining a sequence information from extracted nucleic acids from a biological sample;
   (b) aligning the sequence information (reads) with a reference genome database;

(c) selecting nucleic acid fragments of regulatory regions based on the aligned sequence reads;

(d) producing image data from the selected nucleic acid fragments; and

(e) inputting and analyzing the produced image data to an artificial intelligence model trained to distinguish a normal image from a cancer image, and then comparing an output value with a cut-off value to determine whether or not cancer develops.

2. A method for early diagnosis of cancer based on artificial intelligence, comprising:

(a) obtaining a sequence information from extracted nucleic acids from a biological sample;

(b) aligning the sequence information (reads) with a reference genome database;

(c) selecting nucleic acid fragments of regulatory regions based on the aligned sequence reads;

(d) producing image data from the selected nucleic acid fragments; and

(e) inputting and analyzing the produced image data to an artificial intelligence model trained to distinguish a normal image from a cancer image, and then comparing an output value with a cut-off value, and determining that cancer develops when the output value is higher than the cut-off value.

3. The method according to claim 1 or 2, wherein step (a) to obtain sequence information comprises:

(a-i) obtaining nucleic acids from a biological sample;

(a-ii) removing proteins, fats, and other residues from the obtained nucleic acids using a salting-out method, a column chromatography method, or a bead method to obtain purified nucleic acids;

(a-iii) producing a single-end sequencing or paired-end sequencing library for the purified nucleic acids or nucleic acids randomly fragmented by enzymatic digestion, pulverization, or hydroshearing;

(a-iv) reacting the produced library with a next-generation sequencer; and

(a-v) obtaining sequence information (reads) of the nucleic acids in the next-generation sequencer.

4. The method according to claim 1 or 2, wherein the nucleic acid in step (a) is cell-free DNA.

5. The method according to claim 1 or 2, further comprising:
selecting reads having a mapping quality score of the aligned nucleic acid fragments equal to or greater than a cut-off value prior to step (c).

6. The method according to claim 5, wherein the cut-off value is 50 to 70.

7. The method according to claim 1 or 2, wherein the regulatory region in step (c) is a tissue-specific regulatory region.

8. The method according to claim 7, wherein the tissue-specific regulatory region is **characterized in that** a length and/or amount of cell-free DNA detected for respective tissues is different.

9. The method according to claim 1 or 2, wherein the image in step (d) is a one-dimensional image wherein the x-axis comprises the number of reads for each alignment position of the selected nucleic acid fragment.

10. The method according to claim 1 or 2, wherein the artificial intelligence model in step (e) is an artificial neural network.

11. The method according to claim 10, wherein the artificial neural network is a convolutional neural network (CNN) or a recurrent neural network (RNN).

12. A device for providing information for early diagnosis of cancer based on artificial intelligence, the device comprising:

a decoder configured to extract nucleic acids from a biological sample and decode sequence information;

an aligner configured to align the decoded sequence with a reference genome database;

a nucleic acid fragment selector configured to select nucleic acid fragments of regulatory regions based on the aligned sequence reads;

a data producer configured to produce the selected nucleic acid fragments as image data; and

an information supply configured to input the produced image data to an artificial intelligence model trained to distinguish a normal image from a cancer image, to analyze the data, and to provide information for early diagnosis of cancer.

**13.** A computer-readable storage medium including an instruction configured to be executed by a processor for providing information for early diagnosis of cancer, through the following steps comprising:

(a) obtaining a sequence information from extracted nucleic acids from a biological sample;
(b) aligning the sequence information (reads) with a reference genome database;
(c) selecting nucleic acid fragments of regulatory regions based on the aligned sequence reads;
(d) producing image data from the selected nucleic acid fragments; and
(e) inputting and analyzing the produced image data to an artificial intelligence model trained to distinguish a normal image from a cancer image, and then comparing an output value with a cut-off value, and determining that cancer develops when the output value is higher than the cut-off value.

**14.** A device for early diagnosis of cancer based on artificial intelligence, the device comprising:

a decoder configured to extract nucleic acids from a biological sample and decode sequence information;
an aligner configured to align the decoded sequence with a reference genome database;
a nucleic acid fragment selector configured to select nucleic acid fragments of regulatory regions based on the aligned sequence reads;
a data producer configured to produce the selected nucleic acid fragments as image data; and
a cancer diagnostic unit configured to input the produced image data to an artificial intelligence model trained to distinguish a normal image from a cancer image, to compare an output value with a cut-off value and to determine that cancer develops when the output value is higher than the cut-off value.

**15.** A computer-readable storage medium including an instruction configured to be executed by a processor for conducting early diagnosis of cancer, through the following steps comprising:

(a) obtaining a sequence information from extracted nucleic acids from a biological sample;
(b) aligning the sequence information (reads) with a reference genome database;
(c) selecting nucleic acid fragments of regulatory regions based on the aligned sequence reads;
(d) producing image data from the selected nucleic acid fragments; and
(e) inputting and analyzing the produced image data to an artificial intelligence model trained to distinguish a normal image from a cancer image, and then comparing an output value with a cut-off value, and determining that cancer develops when the output value is higher than the cut-off value.

FIG. 1

FIG. 2

Liver

Blood

Liver

Blood

Liver-specific NFR

Blood-specific NFR

Nucleosome

FIG. 2 (Continued)

## FIG. 3

FIG. 4

FIG. 5

cfDNA reads distribution
at blood-specific NFRs

cfDNA reads distribution
at liver-specific NFRs

Blood-specific NFR

Liver-specific NFR

Human genome(hg19)

cfDNA read

FIG. 6

Input (2000 x 4)

Convolution

BatchNormalization

Activation

Dropout

Prediction

N-times repetition

FIG. 7

AUC using random regions: 0.701
AUC using selected regions: 0.863

## INTERNATIONAL SEARCH REPORT

| International application No. |
| --- |
| **PCT/KR2022/007648** |

**A.    CLASSIFICATION OF SUBJECT MATTER**

**G16H 50/20**(2018.01)i; **G16B 30/10**(2019.01)i; **G16B 40/20**(2019.01)i; **G16H 50/50**(2018.01)i; **G16H 30/40**(2018.01)i; **G16H 30/20**(2018.01)i; **G06N 3/08**(2006.01)i; **G06N 3/04**(2006.01)i

According to International Patent Classification (IPC) or to both national classification and IPC

**B.    FIELDS SEARCHED**

Minimum documentation searched (classification system followed by classification symbols)

G16H 50/20(2018.01); C12M 1/00(2006.01); C12Q 1/68(2006.01); C12Q 1/6886(2018.01); G06F 19/18(2011.01); G06F 19/22(2011.01); G06N 20/00(2019.01); G16B 30/00(2019.01); G16B 30/10(2019.01); G16B 40/00(2019.01); G16B 40/20(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 서열정보(sequence information), 리드(reads), 정렬(alignment), 인공지능(artificial intelligence), 기준값(cut-off value), 핵산단편(nucleic acid fragments), 진단(diagnosis), 암(tumor, metastasis, cancer)

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0085667 A (GREEN CROSS GENOME CORPORATION) 19 July 2019 (2019-07-19)<br>See abstract; and claims 1-15. | 1-15 |
| A | KR 10-1686146 B1 (GREEN CROSS GENOME CORPORATION) 13 December 2016 (2016-12-13)<br>See entire document. | 1-15 |
| A | KR 10-2020-0101106 A (GREEN CROSS GENOME CORPORATION et al.) 27 August 2020 (2020-08-27)<br>See entire document. | 1-15 |
| A | KR 10-2021-0003094 A (CORNELL UNIVERSITY et al.) 11 January 2021 (2021-01-11)<br>See entire document. | 1-15 |
| A | JP 2016-513459 A (INTELLIGENT BIO-SYSTEMS, INC.) 16 May 2016 (2016-05-16)<br>See entire document. | 1-15 |

☑ Further documents are listed in the continuation of Box C.        ☑ See patent family annex.

| | | |
| --- | --- | --- |
| * | Special categories of cited documents: | |
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" | document member of the same patent family |

| Date of the actual completion of the international search | Date of mailing of the international search report |
| --- | --- |
| **01 September 2022** | **01 September 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2022/007648**

### C. DOCUMENTS CONSIDERED TO BE RELEVANT

| Category* | Citation of document, with indication, where appropriate, of the relevant passages | Relevant to claim No. |
|---|---|---|
| A | WO 2021-023650 A1 (INSERM (INSTITUT NATIONAL DE LA SANTE ET DE LA RECHERCHE MEDICALE) et al.) 11 February 2021 (2021-02-11)<br>See entire document. | 1-15 |

Form PCT/ISA/210 (second sheet) (July 2019)

## INTERNATIONAL SEARCH REPORT
### Information on patent family members

International application No.

**PCT/KR2022/007648**

| Patent document cited in search report | | | Publication date (day/month/year) | Patent family member(s) | | | Publication date (day/month/year) |
|---|---|---|---|---|---|---|---|
| KR | 10-2019-0085667 | A | 19 July 2019 | KR | 10-2029393 | B1 | 07 October 2019 |
| | | | | WO | 2019-139363 | A1 | 18 July 2019 |
| KR | 10-1686146 | B1 | 13 December 2016 | | None | | |
| KR | 10-2020-0101106 | A | 27 August 2020 | KR | 10-2381252 | B1 | 01 April 2022 |
| | | | | US | 2022-0148734 | A1 | 12 May 2022 |
| | | | | WO | 2020-171573 | A1 | 27 August 2020 |
| KR | 10-2021-0003094 | A | 11 January 2021 | AU | 2019-228512 | A1 | 03 September 2020 |
| | | | | CA | 3092352 | A1 | 06 September 2019 |
| | | | | CN | 112602156 | A | 02 April 2021 |
| | | | | EP | 3759238 | A1 | 06 January 2021 |
| | | | | JP | 2021-520004 | A | 12 August 2021 |
| | | | | SG | 11202007871 | A | 29 September 2020 |
| | | | | US | 2021-0002728 | A1 | 07 January 2021 |
| | | | | WO | 2019-169044 | A1 | 06 September 2019 |
| JP | 2016-513459 | A | 16 May 2016 | AU | 2014-237067 | A1 | 15 October 2015 |
| | | | | AU | 2014-237067 | B2 | 10 October 2019 |
| | | | | CN | 105474236 | A | 06 April 2016 |
| | | | | CN | 105474236 | B | 25 June 2019 |
| | | | | EP | 2973229 | A1 | 20 January 2016 |
| | | | | EP | 2973229 | B1 | 29 June 2022 |
| | | | | JP | 6809903 | B2 | 06 January 2021 |
| | | | | US | 10249038 | B2 | 02 April 2019 |
| | | | | US | 2014-0267669 | A1 | 18 September 2014 |
| | | | | US | 2017-0132787 | A1 | 11 May 2017 |
| | | | | US | 9591268 | B2 | 07 March 2017 |
| | | | | WO | 2014-150522 | A1 | 25 September 2014 |
| WO | 2021-023650 | A1 | 11 February 2021 | EP | 4007820 | A1 | 08 June 2022 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ULZ, P. ; THALLINGER, G. ; AUER, M. et al.** *Nat. Genet.,* 2016, vol. 48, 1273-1278
- **ULZ, P. et al.** *Nat. Commun.,* 2019, vol. 10, 4666
- **METZKER, M.** *Nature Biotechnology Reviews,* 2010, vol. 11, 31-46